Europäisches Patentamt

(19) European Patent Office    (11) Publication number: **0 307 376**

Office européen des brevets    **A1**

(12) **EUROPEAN PATENT APPLICATION**

(21) Application number: **88850240.8**    (51) Int. Cl.⁴: **A 01 N 63/00**
  **A 61 L 2/18**
(22) Date of filing: **04.07.88**    **//(A01N63/00,59:12,59:00)**

(30) Priority: **10.07.87 SE 8702831**

(43) Date of publication of application:
   **15.03.89 Bulletin 89/11**

(84) Designated Contracting States:
   **AT BE CH DE ES FR GB GR IT LI LU NL SE**

(71) Applicant: **EWOS AKTIEBOLAG**
   **Box 618**
   **S-151 27 Södertälje (SE)**

(72) Inventor: **Knutsson, Maud Inger Christine**
   **Nygard Ashorn**
   **S-619 00 Trosa (SE)**

(74) Representative: **Inger, Lars Ulf Bosson**
   **L + U INGER Patentbyra AB Garvaregatan 12**
   **S-262 63 Ängelholm (SE)**

The title of the invention has been amended (Guidelines for Examination in the EPO, A-III, 7.3).

(54) **Microbiocidal composition.**

(57) The present invention relates to a composition having microbicide effect and comprising iodine and the enzyme lactoperoxidase, whereby it comprises lactoperoxidase in an amount of at least 10 mg/l, a peroxide donor in an amount that gives at least 0.5 mM $H_2O_2$, $I^-$ in a concentration of at least 10 ppm, and a pH adjusting agent in such an amount that pH is 3.25-7.0, when lactoperoxidase is used, and 3.5-6, when horse radish peroxidase is used, preferably 4.5-6.5.

EP 0 307 376 A1

**Description**

## MICROBICIDE COMPOSITION.

Technical field

The present invention relates to a microbicide composition comprising a iodide and the enzyme lactoperoxidase and/or horse radish peroxidase, and hydrogen peroxide and/or a hydrogen peroxide donating system.

The object of the present invention is to obtain a composition, preferably in dry form, which can be used particularly for skin disinfection, but also for other general disinfection purposes to an acceptable cost, and having a low total iodine content, and thereby a less risk for miscolouring and skin irritation, and is a short term composition with an activity term period of some 24 hrs in open air.

Background of the invention

It is previously known to use so called iodophores for skin disinfection and general disinfection, iodophores which are based on a complex between iodine and detergents of the type higher alkyl phenyl polyols or other polymers, such as $PVP-I_2$. Such complexes comprises in order to give a bactericide effect at a relatively large amount of iodine. Due to this fact, and the effect of the detergent and a low pH, they possess an irritating effect on the skin, and thus they have to be replenished with different skin emollient compositions, which partly complicates the product, partly makes it considerably more expensive.

EP-A1-0 175 801 discloses a combined product for disinfecting contact lenses comprising a bactericide having limited time of bacteriological activity, said product comprising peroxidase, a peroxide, and a system of predetermined donor molecules, i.a., iodine salts. The peroxide used is horse radish peroxide. Such a system, will however, as shown below, not work under all conditions, and particularly not at the condition of neutral pH (7). It is said in EP-A1-0 175 801 that its bactericide effect is due to the formation of free radi cals. However, in the tests made and shown herein, not even such an effect can be proved.

It is further known that oxidation of iodide to iodine in the presence of iodate ions ($I^- \text{ -- } I_2$) takes place very rapidly at low pH:es, while it takes place more slowly at pH above 5 and is almost completely stopped at pH 7.

However, in order to maintain a suitable pH, pH has to be above 3, which means that the reaction according to the knowledge known is relatively slow.

It is known from US-A-4,271,149 a composition comprising iodine containing 1000 ppm of $I_2$, and 500-5000 ppm $I^-$ and 50-2000 ppm of iodate at a pH of 5-7.

Description of the present invention.

It has been shown possible to obtain an iodine containing disinfecting composition having microbicide effect, which composition contains lactoperoxidase in a concentration of at least 0.2 mg/l, a hydrogen peroxide donor in an amount which gives a concentration of $H_2O_2$ of at least 0.05 mM, and $I^-$ in a concentration of at least 10 ppm, and a pH adjusting agent in such an amount that pH is 3.25-7.0 when lactoperoxidase is used, preferably 4.5-6.0, and is 3.5-6.0 when horse radish peroxidase is used, preferably 4.5-5.5, and whereby the composition when dissolved in water shows an optical density of at least 0.02 determined at 460 nm.

Further characteristics are evident from the accompanying claims.

Lactoperoxidase has a molecular weight of about 77,000.

10 ppm of $I^-$ correspond to 0.0118 g of NaI or 0.0166 of KI.

The term pH adjusting agent means herein primarily suitable buffer systems, such as citric acid citrate buffer, phosphor ic acid-phosphate buffer and other suitable buffer system which locks pH within the range preferably 4.5-6.5. During the formation of $I_2$ by means of lactoperperoxidase or horse radish peroxidase pH changes considerably, and rapidly, and thus it has to be buffered. It is hereby essential that the composition is buffered so that pH is within the range given for several reasons, viz. partly from a skin irritation point of view, partly from an enzyme activity point of view and from an enzyme stability point of view, but also to obtain a satisfactory microbicide effect.

As a hydrogen peroxide donor different systems can be used, such as enzymatic systems, such as glucose/glucoseoxidase system, xanthin/xanthinoxidase system, solid peroxide donors, such as cupper/ascorbic acid, sodium percarbonate, magnesium peroxide, carbamide peroxide, and $H_2O_2$-solution, alternatively an stabilized $H_2O_2$ emulsion, where stability has been achieved by, e.g., fatty acids. Particularly, $H_2O_2$ solution is used in those cases where the system is reactivated. Generally, a solid peroxide donor is used in those cases where an immediate hydrogen peroxide/$I_2$-formation is requested, while the above enzymatic systems, such as glucose/glucoseoxidase, are used when a more slow, more long term acting effect is requested.

Common milk contains the enzyme lactoperoxidase and an addition of milk should thus be a good source of enzyme. This is however, not the case, as it has turned out that milk inhibits, immediately, the effect of any $I_2$ formed. The enzyme lactoperoxidase is thus added in a purified form, without surplus of organic load.

Thiocyanate, such as sodium thiocyanate, can be added to the composition according to the present invention, which thiocyanate is a known component of the so called LP-system, i.e., the antibacterial system based upon lactoperoxidase, a hydrogen peroxide donor, and thiocyante. It has, however, turned out that thiocyanate can not be added to the present composi tion in higher concentrations than 10 ppm, as one obtains a reduced effect at higher concentrations, unless the thiocyanate is added in such a form that it is released after the formation of $I_2$. Such formulations includes slow release preparations where the thiocyanate containing part is coated with polymer membrane. In this way a first rapid onset will be obtained due to the $I_2$ effect, and then will a more lasting effect be obtained from the conventional lactoperoxidase-thiocyanate-hydrogen peroxide-system.

An advantage using the present system is that it can be reactivated. When $I_2$ is formed by the oxidation of $I^-$ by the influence of hydrogen peroxide the product obtains an microbicide effect. This active $I_2$ is then reduced back to $I^-$ again when it is left and the hydrogen peroxide donor disappears, e.g., by consumption by glucose, or a solid peroxide donor, or decomposition by the enzyme present. However, the system can be readily reactivated by an addition of more lactoperoxidase and/or hydrogen peroxide donor, as $I^-$ is not consumed, or at least is not consumed to any greater extent, provided no organic contamination is present.

It has turned out that the present product has a very rapid onset with regard to microbicide effect, and that one, in certain cases, obtains a considerable killing within a few seconds. The product is thus very well suited for skin disinfection, such as use as an udder disinfectant prior to milking, at general stable hygienic measures, such as cleansing of milking equipment, and disinfection of milk rooms, and in other similar contexts, as well as in dairy cleaning, hospital disinfection, in aquaculture, and the similar where different microorganisms shall be eliminated in large areas to reasonable cost. The product according to the invention is suitable for wound application, as well, where glucose of the plasma is combusted in the use of the glucose/glucoseoxidase system. The composition is thus particularly suitable for diabetics.

The invention will be described more in detail in the follow ing with reference to a number of examples, where comparative examples using active iodophores are used as well.

In Table 1 below the composition of the different compositions are given, pH, LP-content, and absorbancy at 460 nm, which latter is an indirect measure of the $I_2$-content.

In Table 2 the microbicide effect of the different compositions determined on a number of different bacterial strains are given, viz. E. coli Atec 25922, Staph. aureus +alpha+ beta Mj 13151/84, and Strept. agal. S-B 8 after different time intervals. The values given hereby give the number of colony forming units (cfu).

In the Tables below a number of abbreviations have been used, viz:
LP which stands for lactoperoxidase,
HP for horse radish peroxidase,
GOD for glucose oxidase
U/ml for units per milliliter
Iodophor I orig pH stands for a first conventional iodophor present on the market with its original pH (2.74)
Iodophor II orig. pH stands for a second iodophor present on the market with its original pH (4)

TABELL 1.

| Example Composition | 1 | 2 | 3 | 4 | 5 | 6 | 7 | 8 | 9 |
|---|---|---|---|---|---|---|---|---|---|
| LP mg/l | 10 | 10 | 10 | | 20 | 20 | 10 | | |
| HP U/l | | | | 100 | | 100 | | 100 | 100 |
| GOD U/l | 100 | 100 | 100 | 100 | 100 | 100 | 100 | 100 | 100 |
| Glucose % | 0,3 | 0,3 | 0,3 | 0,3 | 0,3 | 0,3 | 0,3 | 0,3 | 0,3 |
| NaSCN ppm | | | | | 40 | 40 | | 6 | |
| MgO$_2$ (25%) ppm | | | | | | | | | |
| Iodide as NaI ppm | 10 | 100 | 100 | 100 | 100 | 100 | 100 | 100 | 100 |
| Urea g/l | 80 | 80 | 20 | 20 | 20 | 20 | | | |
| Carbamide peroxide | | | | | | | | | |
| Citric acid g/l | 0,02 | 0,000048 | | | | | | | |
| Mono-Na-citrate g/l | | | 4,2 | 4,2 | 4,2 | 4,2 | 2,1 | 2,1 | 2,1 |
| Tri-Na-citrate g/l | | | 7,8 | 7,8 | 7,8 | 7,8 | 3,9 | 3,9 | 3,9 |
| pH initially | 5,5 | 6 | | | 5,35 | 5,35 | 5,5 | 5,48 | 5,47 |
| after 30 min | | | | | | | 5,47 | 5,46 | 5,49 |
| 24 hrs | | 5,1 | | | 5,32 | 5,33 | 5,44 | 5,43 | 5,45 |
| 48 hrs | | 5 | | | 5,3 | 5,29 | 5,41 | 5,41 | 5,44 |
| LP determ. U/l | | | | | | | | | |
| after 30 min | | 0,153 | | | 0,42 | 0,56 | 0,16 | 0,21 | 0,3 |
| 24 hrs | | 0,024 | | | 0,28 | 0,49 | 0,03 | 0,21 | 0,24 |
| 48 hrs | | | | | 0,02 | 0,17 | 0,01 | | |
| Absorbancy 460 nm | | | | | | | | | |
| after 10 min | 0,027 | 0,031 | | | 0,004 | 0,004 | 0,028 | 0,048 | 0,09 |
| 24 hrs | | 0,075 | | | 0,003 | 0,003 | 0,055 | 0,051 | 0,041 |
| 48 hrs | | 0,078 | | | 0,045 | 0,045 | 0,028 | 0,041 | 0,014 |

EP 0 307 376 A1

TABELL 1.

| Composition | 10 | 11 | 12 | 13 | 14 | 15 | 16 | 17 | 18 |
|---|---|---|---|---|---|---|---|---|---|
| Example | 10 | 11 | 12 | 13 | 14 | 15 | 16 | 17 | 18 |
| LP mg/l | 10 | 10 | 10 | 10 | 10 | 10 | 10 | 10 | 10 |
| HP U7L | | | | 100 | | | | 100 | |
| GOD U/l | 100 | | 100 | | | | | | |
| Glucose % | 0,3 | | 0,3 | | | | | | |
| NaSCN ppm | 6 | 6 | 6 | 6 | 6 | 6 | | 6 | 6 |
| $MgO_2$ (25%) ppm | | 300 | | 300 | 300 | | | 300 | 300 |
| Iodide as NaI ppm | 100 | 100 | 500 | 1000 | 1000 | 100 | 100 | 100 | 100 |
| Urea g/l | | | | | | | | | |
| Carbamide peroxide | | | | | | 95 | 95 | | |
| Citric acid g/l | | | | | | | | | |
| Mono-Na-citrate g/l | 2,1 | 2,1 | 2,1 | 2,1 | 2,1 | 2,1 | 2,1 | 2,1 | 2,1 |
| Tri-Na-citrate g/l | 3,9 | 3,9 | 3,9 | 3,9 | 3,9 | 3,9 | 3,9 | 3,9 | 3,9 |
| pH initially | | 5,9 | | 5,44 | 5,44 | 5,48 | 5,46 | 5,43 | 5,44 |
| after 30 min | | 5,8(60 min) | | 5,93 | 5,93 | 5,52 | 5,53 | 5,88 | 5,88 |
| 24 hrs | | | | 5,93 | 5,93 | 5,51 | 5,53 | 5,89 | 5,89 |
| 48 hrs | | | | | | | | | |
| LP determ. U/l | | | | | | | | | |
| after 30 min | | 0,011 | | 0,3 | 0,3 | 0,33 | 0,37 | 0,58 | 0,29 |
| 24 hrs | | 0 | | 1,1-0,1 | 1,1-0,1 | 0,001 | 0,002 | 0,08 | 0 |
| 48 hrs | | | | | | | | | |
| Absorbancy 460 nm | | | | | | | | | |
| after 10 min | | 0,24 | | 0,877 | 0,895 | 0,222 | 0,25 | 0,207 | 0,221 |
| 24 hrs | | 0,2 | | 0,699 | 0,697 | 0,208 | 0,239 | 0,194 | 0,203 |
| 48 hrs | | 0,19 | | | | | | | |

EP 0 307 376 A1

TABELL 1.

| Example Composition | 19 | Iodophor I pH orig | Iodophor I pH 5,5 | Iodophor II pH orig | Iodophor II pH 5,5 | 20 | 21 |
|---|---|---|---|---|---|---|---|
| LP mg/l | 1 | | | | | 2.5 | 2.5 |
| HP U/l | | | | | | | |
| GOD U/l | 100 | | | | | | |
| Glucose % | 0,1 | | | | | | |
| NaSCN ppm | 6 | | | | | | |
| $MgO_2$ (25%) ppm | | | | | | | |
| Iodide as NaI, ppm | 60 | 60 | 60 | 60 | 60 | 60 | 60 |
| Urea g/l | | | | | | | |
| Carbamide peroxide | 9,5 | | | | | 94 | 94 |
| Citric acid g/l | 0,05 mM | | | | | 0.005M | 0.055M |
| Mono-Na-citrate g/l | pH 5,5 | | | | | pH 5.5 | pH 3.5 citrate buffer |
| Tri-Na-citrate g/l | citrate buffer | | | | | citrate buffer | |
| pH initially | 5,5 | 2,74 | 5,4 | 4 | 5,5 | 5.07 | 3.64 |
| after 30 min | 5,6 | 2,75 | 5,5 - | 3,8 | 5,5 | | |
| 24 hrs | 5,6 | 2,95 | 5,5 | 3,8 | 5,5 | 5.1 | 3.6 |
| 48 hrs | | | | | | 5.1 | 3.6 |
| LP determ. U/l | | | | | | | |
| after 30 min | 0,018 | | | | | | |
| 24 hrs | | | | | | | |
| 48 hrs | | | | | | | |
| Absorbancy 460 nm | | | | | | | |
| after 10 min | 0,017 | 0,444 | 0,546 | 0,697 | 0,820 | | |
| 24 hrs | 0,00 | 0,404 | 0,516 | 0,677 | 0,791 | | |
| 48 hrs | 0,04 | | | | | | |

TABELL 2.

| Example<br>Bacteria | 1 | 2 | 3 | 4 | 5 | 6 | 7 | 8 | 9 |
|---|---|---|---|---|---|---|---|---|---|
| E.coli inoc. | $9,7 \times 10^6$ | $1,7 \times 10^6$ | $1,6 \times 10^7$ | $1,6 \times 10^7$ | $6,6 \times 10^6$ | $6,6 \times 10^6$ | | | |
| 30 sek | $7,0 \times 10^1$ | <1 | <1 | <1 | $>10^6$ | $>10^6$ | | | |
| 2 min | <1 | <1 | <1 | <1 | $>10^6$ | $>10^6$ | | | |
| 10 min | <1 | <1 | <1 | <1 | | | | | |
| 60 min | <1 | <1 | <1 | <1 | <1 | <1 | | | |
| 120 min | | | | | | | | | |
| 240 min | | | | | <1 | <1 | | | |
| Staph.aureus inoc. | $1,8 \times 10^6$ | $2,9 \times 10^6$ | $2,4 \times 10^7$ | $2,4 \times 10^7$ | $1,9 \times 10^7$ | $1,9 \times 10^7$ | $5,5 \times 10^7$ | $3,7 \times 10^7$ | $3,7 \times 10^7$ |
| 30 sek | $>10^7$ | $>10^7$ | <1 | <1 | $>10^7$ | $>10^7$ | | | |
| 2 min | $1,8 \times 10^6$ | <1 | <1 | <1 | $>10^7$ | $>10^7$ | | | |
| 10 min | <1 | <1 | <1 | <1 | | | 30 | <10 | <10 |
| 60 min | <1 | <1 | <1 | <1 | $>10^7$ | $>10^7$ | <10 | <10 | <10 |
| 120 min | | | | | | | <10 | <10 | <10 |
| 240 min | | | | | $3,2 \times 10^4$ | $7,2 \times 10^6$ | | | |
| Strep/Staph[1] inoc. | $2,2 \times 10^6$ | $1,5 \times 10^6$ | | | $5,3 \times 10^6$ | $5,3 \times 10^6$ | $3,0 \times 10^7$ | $2,8 \times 10^7$ | $2,8 \times 10^7$ |
| 30 sek | <1 | 10 | | | $>10^6$ | $>10^6$ | | | |
| 2 min | <1 | 90 | | | $>10^6$ | $>10^6$ | | | |
| 10 min | <1 | <1 | | | | | <10 | <10 | <10 |
| 60 min | <1 | <1 | | | $2 \times 10^2$ | $3 \times 10^2$ | <10 | <10 | <10 |
| 120 min | | | | | | | <10 | <10 | <10 |
| 240 min | | | | | <1 | <1 | | | |

1)
    Strep. agal S-B 8 has been used for Examples 1 and 2. Strep. uberis has been used for Examples 5 and 6. Staph. aureus has been used for Examples 7-9, whereby the solutions were 29 hrs old. The solutions 1-6 were 1 hr old.

EP 0 307 376 A1

TABELL 2.

| Example Bacteria | 10 | 11 | 12 | 13 | 14 | 15 | 16 | 17 | 18 |
|---|---|---|---|---|---|---|---|---|---|
| E.coli inoc. | | | | | | | | | |
| 30 sek | | | | | | | | | |
| 2 min | | | | | | | | | |
| 10 min | | | | | | | | | |
| 60 min | | | | | | | | | |
| 120 min | | | | | | | | | |
| 240 min | | | | | | | | | |
| Staph.aureus inoc. | $5,6 \times 10^7$ | $6,2 \times 10^8$ | $5,6 \times 10^7$ | $3,8 \times 10^7$ | $3,8 \times 10^7$ | $4,9 \times 10^7$ | $4,9 \times 10^7$ | $4,5 \times 10^7$ | $4,5 \times 10^7$ |
| 30 sek | | | | <10 | $4,4 \times 10^2$ | 50 | $2,9 \times 10^2$ | 10 | $6,5 \times 10^2$ |
| 2 min | | | | <10 | <10 | <10 | 40 | <10 | <10 |
| 10 min | 70 | <10 | | <10 | <10 | <10 | <10 | <10 | <10 |
| 60 min | <10 | <10 | <10 | <10 | <10 | <10 | <10 | <10 | <10 |
| 120 min | | | | | | | | | |
| 240 min | $2,0 \times 10^3$ | <10 | <10 | | | | | | |
| S.aureus [2] inoc. | $3,8 \times 10^7$ | $7,7 \times 10^6$ | $3,8 \times 10^7$ | $3,8 \times 10^7$ | $3,8 \times 10^7$ | $4,9 \times 10^7$ | $4,9 \times 10^7$ | $3,1 \times 10^7$ | $3,1 \times 10^7$ |
| 30 sek | | | | $1,1 \times 10^2$ | <10 | $6,0 \times 10^2$ | $2,0 \times 10^3$ | <10 | 20 |
| 2 min | | | | 20 | <10 | <10 | 100 | <10 | <10 |
| 10 min | <10 | <10 | <10 | <10 | <10 | <10 | <10 | <10 | <10 |
| 60 min | <10 | <10 | 60 | <10 | <10 | <10 | <10 | <10 | <10 |
| 120 min | | | | | | | | | |
| 240 min | <10 | <10 | $1,2 \times 10^3$ | | | | | | |

[2] The solutions of the compositions used were 29 hrs old, with the exception for the solutions 13-18. which were 5 hrs old

TABELL 2.

| Example | 19 | Iodophor I pH orig | Iodophor I pH 5,5 | Iodophor II pH orig | Iodophor II pH 5,5 | 20 | 21 |
|---|---|---|---|---|---|---|---|
| Bacteria | | | | | | | |
| E.coli inoc. | | | | | | | |
| 30 sek | | | | | | | |
| 2 min | | | | | | | |
| 10 min | | | | | | | |
| 60 min | | | | | | | |
| 120 min | | | | | | | |
| 240 min | | | | | | | |
| Staph.aureus inoc. | $3,8 \times 10^7$ | $3,6 \times 10^7$ | $3,6 \times 10^7$ | $4,8 \times 10^7$ | $4,8 \times 10^7$ | $3.9 \times 10^7$ | $3.9 \times 10^7$ |
| 30 sek | $>10^7$ | 590 | $2,0 \times 10^3$ | $4,9 \times 10^4$ | $1,7 \times 10^7$ | $3.6 \times 10^2$ | $1.3 \times 10^3$ |
| 2 min | $>10^7$ | <10 | 100 | 30 | $1,1 \times 10^5$ | <10 | <10 |
| 10 min | $>10^7$ | <10 | <10 | <10 | 10 | <10 | <10 |
| 60 min | 20 | <10 | 200 | <10 | $4,0 \times 10^3$ | <10 | <10 |
| 120 min | | | | | | | |
| 240 min | | | | | | | |
| Staph.aureus inoc. [2] | $4,9 \times 10^7$ | $2,8 \times 10^7$ | $2,8 \times 10^7$ | $3,1 \times 10^7$ | $3,1 \times 10^7$ | | |
| 30 sek | $>10^7$ | $3,0 \times 10^3$ | $4,0 \times 10^4$ | <10 | $2,7 \times 10^4$ | | |
| 2 min | $>10^7$ | <10 | 500 | <10 | 170 | | |
| 10 min | $1,6 \times 10^4$ | <10 | <10 | <10 | 600 | | |
| 60 min | $1,8 \times 10^3$ | <10 | <10 | <10 | 600 | | |
| 120 min | | | | | | | |
| 240 min | | | | | | | |

[2] The solutions of the compositions used were 24 hrs old

EP 0 307 376 A1

As evident from Tables 1 and 2 a considerable microbicide effect is obtained by means of the compositions according to the present invention. The remarkable thing is that they functions well at a slightly acidic, almost neutral pH, at which pH iodophores do not function, when pH has been adjusted to substantially neutral value. In the latter case the microbicide effect has almost completely failed.

The present composition is present in a water free form, at least concerning the hydrogen peroxide donating/formating part. Thus the composition according to the invention is present preferably in dry form, but can also be present in the form of a paste which comprises two parts which are brought together at the use. Even a liquid composition can be used considering the basic demand, viz that the hydrogen peroxide donating/formating part is kept out of contact with water until in use. Besides a pure dry pulverulent mixture, the composition can be present in the form of tablets and granules as well as double layer tablets which are dissolved in a suitable amount of water prior to use.

The following Examples 22-31 were prepared for comparative testing. Thus compositions according to the present invention were prepared, as well as according to EP-A1-0 175 801. Example 24 is prepared in accordance with Example 5 of EP-A1-0 175 801, and Example 25 is the same as Ex. 24 but for a weaker buffer, 0.01M. Example 27 is the same as Ex. 24 except for the concentration of the buffer and the pH.

Table 3 below gives the different compositions of Examples 22-31.

Table 4 below gives the absorbancy at 460 nm for each of the solutions of Ex. 22-31.

Following the UV absorbancy test four compositions were picked out for a bactericide test, viz. the compositions of Ex. 22, 24, 26, and 27, respectively, whereby the compositions were tested agaisnt Staph. aureus MJ 13151/84, Staph. aureus 1243/87, Pseudomonas aeruginosa Ps. q. 41, and E. coli A126, respectively, with regard to their bactericide effect. The Staph. aureus strains were inocculated in an amount of $8 \times 10^7$ cfu/ml, Ps aeruginosa in an amount of $1.1 \times 10^8$ cfu/ml, and E. coli in an amount of $6.8 \times 10^7$ cfu/ml. Surviving cfu were tested for after 30 s, 60 s, 2 min, and 10 min, respectively. The bactericide effect obtained is shown in Table 5 below, whereby the killing effect is given in percentage of inhibition in a log scale. Zero value indicates no or very weak killing capacity, while 100% corresponds to total killing.

## Table 3.

| Components | 22 | 23 | 24 | 25 | 26 | 27 | 28 | 29 | 30 | 31 |
|---|---|---|---|---|---|---|---|---|---|---|
| LP | 10 | 10 | | | 10 | | 10 | 10 | | |
| HP | | | 10 | 10 | | 10 | | | 10 | 10 |
| Phosphate buffer | 0.1 | 0.01 | 0.1 | 0.01 | | | 0.1 | 0.01 | 0.1 | 0.01 |
| Citrate buffer | | | | | 0.01 | 0.01 | | | | |
| NaI, mM | 0.05 | 0.05 | 0.05 | 0.05 | 0.05 | 0.05 | 0.05 | 0.05 | 0.05 | 0.05 |
| $H_2O_2$, mM | 0.1 | 0.1 | 0.1 | 0.1 | 0.1 | 0.1 | | | | |
| Urea peroxide | | | | | | | 0.1 | 0.1 | 0.1 | 0.1 |
| pH | 7.0 | 7.0 | 7.0 | 7.0 | 5.0 | 5.0 | 7.0 | 7.0 | 7.0 | 7.0 |

Table 4.

| Example | Absorbancy at 460 nm | | | | pH | |
|---|---|---|---|---|---|---|
| | initially | 10 min | 30 min | 24 hrs | initially | 30 min |
| 22 | 0.065 | 0.063 | 0.056 | 0.018 | 7.0 | 7.0 |
| 23 | 0.104 | 0.100 | 0.091 | 0.035 | 7.1 | 7.1 |
| 24 | 0.000 | 0.000 | 0.002 | 0.000 | 7.0 | 7.1 |
| 25 | 0.000 | 0.000 | 0.001 | 0.000 | 7.1 | 7.1 |
| 26 | 0.171 | 0.163 | 0.160 | 0.078 | 5.1 | 5.1 |
| 27 | 0.183 | 0.175 | 0.167 | 0.070 | 5.1 | 5.1 |
| 28 | 0.052 | 0.048 | 0.047 | 0.011 | 7.0 | 7.0 |
| 29 | 0.065 | 0.061 | 0.059 | 0.022 | 7.1 | 7.1 |
| 30 | 0.003 | 0.007 | 0.008 | 0.003 | 7.0 | 7.0 |
| 31 | 0.004 | 0.007 | 0.014 | 0.005 | 7.0 | 7.0 |

Table 4.

## Table 5.

| Example | 22 | 24 | 26 | 27 |
|---|---|---|---|---|
| S. aureus /84 | | | | |
| 30 s | 0 | 0 | 100 | 77.1 |
| 60 s | 0 | 0 | 100 | 100 |
| 2 min | 40.3 | 0 | 100 | 100 |
| 10 min | 66.9 | 0 | 100 | 100 |
| S. aureus /87 | | | | |
| 30 s | 0 | 0 | 100 | 100 |
| 60 s | 0 | 0 | 100 | 100 |
| 2 min | 0 | 0 | 100 | 100 |
| 10 min | 28.5 | 0 | 100 | 100 |
| P. aeruginosa | | | | |
| 30 s | 66.4 | 0 | 100 | 100 |
| 60 s | 73.7 | 0 | 100 | 100 |
| 2 min | 100 | 0 | 100 | 100 |
| 10 min | 100 | 0 | 100 | 100 |
| E. coli | | | | |
| 30 s | 58.3 | 0 | 79.5 | 64.1 |
| 60 s | 63.3 | 0 | 100 | 100 |
| 2 min | 77.3 | 0 | 100 | 100 |
| 10 min | 100 | 0 | 100 | 100 |

As evident from the Tables above, particularly Table 5, the composition of EP-A1-0 175 801, Ex. 5, does not work under the present conditions. It is also evident that the compositions of the present invention fulfill the object of having a rapid onset and good killing of bacteria.

**Claims**

1. Microbicide composition comprising iodide and lactoperoxidase and/or horse radish peroxidase, and a hydrogen peroxide donor, suitable in substantially water free form, characterized in that it comprises lactoperoxidase, or corresponding horse radish peroxidase, in an amount of at least 0.2 mg/l, a peroxide donor in an amount that gives a concentration of $H_2O_2$ of at least 0.05 mM, and I⁻ in a concentration of at least 10 ppm, and a pH adjusting agent in such an amount that pH is 3.25-7.0, preferably 4.5-6.0, when lactoperoxidase is used, and is 3.5-6.0, preferably 4.5-5.5, when horse radish peroxidase is used, and whereby the optical density of an aqueous solution of the composition determined at 460 nm is at least 0.02 at the dissolution in water.

2. Composition according to claim 1, characterized in that the amount of lactoperoxidase is 2 mg/l.

3. Composition according to claims 1-2, characterized in that it further comprises thiocyanate in an amount of at most 10 ppm, unless present in a such a form that it is released after the formation of $I_2$.

4. Composition according to claim 1, characterized in that the peroxide donor is glucose/glucoseoxidase.

5. Composition according to claim 1, characterized in that the peroxide donor is magnesium peroxide.

6. Composition according to claim 1, characterized in that the peroxide donor is carbamide peroxide.

7. Composition according to one or more of claims 1-6, characterized in that the amount of $I^-$ is at least 50 ppm.

8. Composition according to claim 1, characterized in that pH is 5.0 to 6.0.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-4 282 324 (S.L. NEIDLEMAN et al.) * Column 2, line 38 - column 3, line 9; column 3, lines 25-32; examples; claims * | 1-8 | A 01 N 63/00 A 61 L 2/18 // (A 01 N 63/00 A 01 N 59:12 A 01 N 59:00 ) |
| X | CHEMICAL ABSTRACTS, vol. 68, no. 7, 12th February 1968, page 2665, no. 27662h, Columbus, Ohio, US; J.K. SEYMOUR: "Iodination of bacteria: a bactericidal mechanism", & J. EXP. MED. 126(6), 1063-78(1967) * Abstract * | 1-8 | |
| A | CHEMICAL ABSTRACTS, vol. 99, no. 23, 5th December 1983, page 458, no. 191505v, Columbus, Ohio, US; A.M. SUGAR et al.: "Susceptibility of Blastomyces dermatitidis strains to products of oxidative metabolism", & INFECT. IMMUN. 1983, 41(3), 908-12 * Abstract * | 1-8 | |
| A | CHEMICAL ABSTRACTS, vol. 89, no. 11, 11th September 1978, page 86, no. 85242r, 85243s, Columbus, Ohio, US; E.L. THOMAS et al.: "Cofactor role of iodide in peroxidase antimicrobial action against Escherichia coli", & ANTIMICROB. AGENTS CHEMOTHER. 1978, 13(6), 1000-5; "Oxidation of Escherichia coli sulfhydryl components by the peroxidase-hydrogen peroxide-iodide antimicrobial system", & ANTIMICROB. AGENTS CHEMOTHER. 1978, 13(6), 1106-10 * Abstracts *            -/- | 1-8 | TECHNICAL FIELDS SEARCHED (Int. Cl.4) A 01 N A 61 L |

The present search report has been drawn up for all claims

| Place of search | Date of completion of the search | Examiner |
|---|---|---|
| THE HAGUE | 27-10-1988 | FLETCHER A.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | EP-A-0 175 801 (J. KESSLER et al.)<br>* Claims *<br>----- | 1-8 | |
| | | | TECHNICAL FIELDS SEARCHED (Int. Cl.4) |

The present search report has been drawn up for all claims

| Place of search | Date of completion of the search | Examiner |
|---|---|---|
| THE HAGUE | 27-10-1988 | FLETCHER A.S. |